# EUROPÄISCHE PATENTSCHRIFT

(11) **EP 0 658 536 B1**
(45) Veröffentlichungstag und Bekanntmachung des Hinweises auf die Patenterteilung: **24.09.1997**
(21) Anmeldenummer: 94119044.9
(22) Anmeldetag: 02.12.1994
(51) Int. Cl.: C07C 68/08, C07C 69/96

(54) **Verfahren zur Abtrennung von Methanol aus einem Gemisch von Dimethylcarbonat und Methanol**
Process for the separation of methanol from a mixture of dimethyl carbonate and methanol
Procédé de séparation du méthanol d'un mélange de carbonate de diméthyle et de méthanol

(30) Priorität: 15.12.1993 DE 4342713
(43) Veröffentlichungstag der Anmeldung: 21.06.1995
(73) Patentinhaber: BAYER AG, 51368 Leverkusen (DE)
(72) Erfinder: Janisch, Dr. Ingo, D-51515 Kürten (DE); Landscheidt, Dr. Heinz, D-47057 Duisburg (DE); Strüver, Dr. Werner, D-51375 Leverkusen (DE); Klausener, Dr. Alexander, D-50670 Köln (DE)

(56) Entgegenhaltungen:
- US-A- 4 582 645

## Beschreibung

Bei der technischen Herstellung von Dimethylcarbonat (DMC) fallen häufig Gemische von DMC und Methanol an, die gegebenenfalls noch weitere Bestandteile enthalten können. So wird beispielsweise in EP 413 215 ein Verfahren zur Herstellung von DMC beschrieben, bei dem DMC im Gemisch mit Methanol und Wasser entsteht.

Zur Verwendung von DMC als Reagenz für weitere Umsetzungen wie der Umesterung zu anderen Carbonaten, vor allem zu Diphenylcarbonat oder anderen Diaryl-, aber auch Arylalkyl-, Dialkyl- und Dialkenylcarbonaten, ist es jedoch erforderlich, es in weitgehend reiner Form, insbesondere weitgehend frei von Methanol, einsetzen zu können. Zu diesem Zweck ist ein leistungsfähiges Verfahren zur möglichst vollständigen Abtrennung von anderen Stoffen, insbesondere von Methanol, von Gemischen von DMC mit solchen anderen Stoffen, von hohem Interesse.

Bei zahlreichen Verwendungen von DMC, beispielsweise für die Durchführung von Umesterungsreaktionen, insbesondere für die Herstellung von Mono- und Diarylcarbonaten, gelingt es nicht, DMC vollständig zum Umsatz zu bringen. Es fallen bei derartigen Reaktionen neben den gewünschten Reaktionsprodukten Gemische an, die außer dem freigesetzten Methanol zum Teil noch beachtliche Mengen des unumgesetzten Wertstoffes DMC enthalten.

Eine Abtrennung von Methanol aus solchen Gemischen in möglichst reiner Form erlaubt zum einen seine erneute Bereitstellung für eine wirtschaftliche Nutzung, z.B. zum Zwecke der Rückführung in den Herstellungsprozeß für DMC. Zum anderen werden die in derartigen Gemischen vorhandenen Restmengen an DMC in verwertbar angereicherter Form zurückgewonnen, was einen weiteren ökonomischen Vorteil darstellt.

Zusammenfassend läßt sich daher sagen, daß insbesondere im Sinne eines möglichen Einsatzes von DMC als Ersatzstoff für das hochgiftige Phosgen nicht nur ein ökonomisch befriedigendes Herstellverfahren, sondern auch ein effizient arbeitendes Trennverfahren für die oben genannten Gemische aus Methanol und DMC und gegebenenfalls noch weiteren Komponenten zur Verfügung stehen muß.

Destillativ lassen sich die genannten Gemische nur unter hohem Aufwand, d.h. nicht auf einfache Weise, trennen, da DMC mit Methanol bei Normaldruck ein Azeotrop der ungefähren Zusammensetzung 70 % Methanol, 30 % DMC bildet.

Eine Auftrennung derartiger Gemische durch Ausnutzen der Druckabhängigkeit der Azeotropzusammensetzung, etwa durch Destillieren bei höheren oder tieferen Drücken als bei Normaldruck oder durch sukzessives Destillieren bei höheren und tieferen Drücken als bei Normaldruck (Zweidruck-Destillation), wie dies beispielsweise in JP 02 212 456 (zitiert nach CA 114, 81029) beschrieben wird, ist mit hohen Energie- und Investitionskosten verbunden.

Zur Durchführung einer destillativen Auftrennung von Gemischen aus Methanol und DMC wurde auch vorgeschlagen, die Destillation unter Zusatz weiterer Hilfsstoffe vorzunehmen. So soll nach DE-OS 2 737 265 der Zusatz von Kohlenwasserstoffen, wie n-Heptan, eine Trennung beider Komponenten auf dem Weg einer Azeotropdestillation ermöglichen; nach DE-OS 2 706 684 gelingt die destillative Auftrennung von Gemischen aus DMC und Methanol unter Zusatz von organischen Lösungsmitteln, wie Methylglycolacetat, auf dem Wege einer Extraktivdestillation. Bei beiden Varianten besteht die Notwendigkeit, den Hilfsstoff abzutrennen und im Kreis zu führen. Darüber hinaus kann durch die Anwesenheit weiterer Stoffe die Reinheit des Dimethylcarbonats beeinträchtigt werden.

Neben diesen thermischen Verfahren wurden auch andere Verfahren zur Abtrennung von Methanol aus Gemischen mit DMC vorgeschlagen. So wird beispielsweise in US 4.960.519 die Pervaporation an speziellen Membranen beschrieben. Diese Trenntechnik erfordert jedoch im allgemeinen eine Verschaltung mit zusätzlichen destillativen Trennoperationen und ist aufgrund der aufwendigen zum Einsatz kommenden Apparaturen mit hohen Kosten verbunden, die eine Nutzung im industriellen Bereich fraglich erscheinen lassen.

Ferner wurde in US 4 582 645 vorgeschlagen, DMC aus Gemischen durch Absorption an Zeolithen zu entfernen. Aufgrund der begrenzten Aufnahmekapazität der Zeolithe (100 mg DMC/2,5 g Zeolith) lassen sich auf diese Weise nur kleine Mengen DMC abtrennen. Dieses Verfahren kommt daher nur für Gemische in Betracht, deren DMC-Anteil gering ist.

Es bestand demnach die Aufgabe, ein Verfahren zu finden, daß die Abtrennung von Methanol von Gemischen mit DMC auf wirtschaftlich sinnvolle Weise ermöglicht.

Überraschenderweise wurde gefunden, daß durch Behandlung von DMC und Methanol enthaltenden Gemischen mit Ionenaustauscherharzen Methanol aus diesen Gemischen selektiv entfernt wird, so daß man weitgehend methanolfreies DMC von hoher Reinheit erhält. Der Methanolgehalt beträgt 0,01 - 40 Gew.-%, bezogen auf das Gewicht des Gemisches.

Das erfindungsgemäße Verfahren eignet sich grundsätzlich auch für solche Methanol und Dimethylcarbonat enthaltenden Gemische, die gegebenenfalls noch weitere Stoffe enthalten. Bevorzugt werden Gemische eingesetzt deren Methanolgehalt maximal 30 Gew.-%, besondern bevorzugt solche, deren Methanolgehalt maximal 10 Gew.-% beträgt.

Die Erfindung betrifft ein Verfahren zur Abtrennung von Methanol aus einem Gemisch von Dimethylcarbonat (DMC) und Methanol, in welchem der Methanolgehalt 0,01 - 40 Gew.-%, bezogen auf das Gewicht der Mischung beträgt, das dadurch gekennzeichnet ist, daß ein solches Gemisch mit einem Ionenaustauscher in einer Menge von 1-100 % des Gewichts des Gemisches bei -50°C bis +100°C und 0,1-10 bar behandelt wird.

In einer Ausführungsform des Verfahrens wird das DMC/Methanol-Gemisch, das gegebenenfalls noch weitere Stoffe enthalten kann, mit Ionenaustauscherharz versetzt. Nach einer zur Absorption des Methanols durch das betreffende Ionenaustauscherharz ausreichenden Zeit, die z.B. durch gaschromatographische Analyse der überstehenden Flüssigkeit in bestimmten zeitlichen Abständen ermittelt werden kann, wird das Harz durch Filtration mechanisch abgetrennt. Dabei beträgt die Menge des eingesetzten Ionenaustauscherharzes im allgemeinen 1-100 %, bevorzugt 5-50 % des Gewichtes des von Methanol zu befreienden Gemisches.

In einer anderen Ausführungsform wird ein Strom des zu behandelnden DMC/Methanol-Gemisches, das gegebenenfalls noch weitere Bestandteile, wie sie beispielsweise bei der Herstellung von DMC entstehen können, enthalten kann, über eine Säule, die mit Ionenaustauscherharz gefüllt ist, geleitet. Dabei beträgt die Belastung des Ionenaustauschers 1-100 Volumeneinheiten des zu trennenden Gemisches pro Volumeneinheit des Ionenaustauscherharzes und Stunde, was im wesentlichen dem obigen Gewichtsverhältnis entspricht. Hierbei kann in einer quasikontinuierlichen Arbeitsweise verfahren werden, indem eine Säule bis zu ihrer Erschöpfung benutzt wird und anschließend auf eine frische Säule geschaltet wird, während die erste regeneriert wird.

Das erfindungsgemäße Verfahren kann bei Temperaturen von -50 bis 100°C, bevorzugt bei -20 bis 100°C, besonders bevorzugt bei 0-50°C, durchgeführt werden.

Das erfindungsgemäße Verfahren kann in einem Druckbereich von 0,1 bis 10 bar durchgeführt werden.

Als Absorberharze, die für die Durchführung des erfindungsgemäße Verfahren in Frage kommen, seien beispielsweise synthetische Ionenaustauscher mit Kationen oder Anionen austauschenden Gruppen oder Gemische solcher Ionenaustauscher genannt.

Solche Ionenaustauscher haben beispielsweise eine Matrix auf der Basis vernetzter Styrolpolymere. Als Vernetzer seien beispielsweise Divinylbenzol, Trivinylbenzol oder Trivinylcyclohexan in einer Menge von 1-80 Gew.-%, bezogen auf die Gesamtmenge der Comonomeren, genannt.

Die Matrix kann jedoch gegebenenfalls auch ein Phenol-Formaldehyd-Kondensat, ein Acrylharz, ein Methacrylharz oder ein Epichlorhydrin-Polyamin-Kondensat, jeweils in vernetzter Form mit obigen Vernetzern, sein. Solche vernetzten Matrizes können in der gelförmigen oder der makroporösen Form eingesetzt werden.

Als funktionelle Gruppen der Kationenaustauscher seien beispielsweise genannt: Sulfonsäuregruppen, Phosphonsäuregruppen und Carboxylgruppen, jeweils in der H⁺-Form oder in der Metallionen- bzw. Kationen-Form.

Als in diesem Sinne in Frage kommende Metallionen bzw. Kationen seien beispielhaft genannt: Li⁺, Na⁺, K⁺, Rb⁺, Cs⁺, NH₄⁺, Cu²⁺, Ag⁺, Mg²⁺, Ca²⁺, Ba²⁺, Zn²⁺, Al³⁺, Sn²⁺, Pb²⁺, Ce⁴⁺, UO₂²⁺, Cr³⁺, Co²⁺, Ni²⁺, Fe²⁺, Fe³⁺ oder Pd²⁺.

Funktionelle Gruppen der Anionenaustauscher können beispielsweise sein: stickstoffhaltige funktionelle Gruppen des Typs -NR₃⁺, wie beispielsweise -N(CH₃)₃⁺ oder -N⁺(CH₃)₂CH₂CH₂OH, weiterhin stickstoffhaltige funktionelle Gruppen des Typs -NR₂, wie beispielsweise -N(CH₃)₂, sowie N-Oxid-Gruppen. Solche stickstoffhaltigen funktionellen Gruppen können als austauschbares Gegenion beispielsweise Ionen aus der Gruppe OH⁻, Cl⁻ und SO₄²⁻ enthalten. R kann außer CH₃ und CH₂CH₂OH auch Ethyl, Propyl, Isopropyl, Butyl, Isobutyl, Benzyl oder Phenyl bedeuten, von denen auch verschiedene an einem N-Atom auftreten können.

Ionenaustauscher der beschriebenen Art haben beispielsweise Totalkapazitäten für den Ionenaustausch von etwa 0,2-6 val/l. Solche Harze und ihre Herstellungsverfahren sind seit langem bekannt (Ullmanns Enzyklopädie der technischen Chemie, 4. Aufl./Band 13, S. 279 ff., Verlag Chemie, 1977).

Beispiele für erfindungsgemäß einsetzbare Harze sind: Sulfonsäuregruppen enthaltende gelförmige Styrol-Divinylbenzolharze, Sulfonsäuregruppen enthaltende makroporöse Styrol-Divinylbenzolharze, Carboxylgruppen enthaltende gelförmige oder makroporöse Acrylsäure-Divinylbenzolharze, gelförmige oder makroporöse Styrol-Divinylbenzolharze mit -N(CH₃)-Gruppen, schwachbasische, makroporöse Harze des Acrylamid-Divinylbenzol-Typs, stark basische, makroporöse Harze des Acrylamid-Divinylbenzol-Typs oder Sulfonsäuregruppen enthaltende vernetzte Phenol-Formaldehydharze.

Diese Aufzählung ist nicht vollständig und stellt keine Einschränkung auf die genannten Harze dar.

Viele der genannten Harze sind Handelsprodukte verschiedener Hersteller.

In bevorzugter Weise werden Kationenaustauscher, besonders bevorzugt in einer mit Metallkationen beladenen Form, eingesetzt.

Die im Zuge des erfindungsgemäßen Verfahrens anfallenden methanolhaltigen Ionenaustauscherharze lassen sich problemlos durch Erhitzen im Vakuum oder Gasstrom regenerieren.

Das hierbei wieder freiwerdende Methanol kann entweder durch Kühlung aufgefangen werden, oder die erhaltenen methanolhaltigen Gasströme können zu chemischen Reaktionen, bevorzugt zur Herstellung von DMC, eingesetzt werden.

### Beispiele

### Beispiel 1

Wasserfeuchtes, gelförmiges sulfonsäuregruppenhaltiges Styrol-Divinylbenzol(DVB)-Polymerisat (Handelsprodukt Lewatit® SC 104 der Bayer AG) wurde bei 80°C und 250 mbar 24 h lang getrocknet.

Zu einer Mischung aus 95 g Dimethylcarbonat und 5 g Methanol wurden 10 g des dermaßen vorbehandelten Austauscherharzes gegeben. Man ließ bei Raumtemperatur rühren. Nach 30 min wurde filtriert und das Filtrat gaschromatographisch untersucht.

Danach betrug der Gehalt des DMC im Filtrat über 98 %.

### Beispiele 2 bis 7

Die in Tabelle 1 aufgeführten Harze wurden jeweils wie in Beispiel 1 vorbehandelt. Die weitere Versuchsdurchführung erfolgte ebenfalls, wie in Beispiel 1 beschrieben. Es sind jeweils die Ausgangskonzentrationen der Dimethylcarbonat/Methanol-Gemische und die erhaltenen Konzentrationen des Filtrats nach 3 h Kontaktzeit angegeben.

**Tabelle 1**

| Beispiel | Austauscherharz | Bezeichnung | DMC-Konzentration | |
|---|---|---|---|---|
| | | | Feed | Filtrat |
| 2 | gelförmig sulfonsäuregruppenhaltig | Bayer-Katalysator K 1131® | 95 % | 97,9 % |
| 3 | gelförmig sulfonsäuregruppenhaltig | Bayer-Katalysator K 1131® | 90 % | 94, 1 % |
| 4 | gelförmig sulfonsäuregruppenhaltig | stark saurer Ionenaustauscher mit 4 % Vernetzer (DVB) | 90 % | 94,0 % |
| 5 | gelförmig sulfonsäuregruppenhaltig | stark saurer Ionenaustauscher mit 3 % Vernetzer (DVB) | 90 % | 94,1 % |
| 6 | makroporös trimethylammoniumgruppenhaltig | Lewatit® MP 500 | 90 % | 92,5 % |
| 7 | gelförmig sulfonsäuregruppenhaltig | Bayer-Katalysator K 1131® mit K⁺ beladen | 90 % | 91,2 % |

### Beispiel 8

Eine Mischung aus 95 g Dimethylcarbonat und 5 g Methanol wurde innerhalb von 3 h über eine Säule geleitet, die 10 g gelförmiges sulfonsäuregruppenhaltiges Styrol-Divinylbenzol-Polymerisat (Handelsprodukt K 1131® dei Bayer AG) enthielt.

Das aufgefangene Eluat wurde gaschromatographisch analysiert.

Nach 1 h hatte das Eluat eine Zusammensetzung von 99,8 % Dimethylcarbonat und 0,2 % Methanol.

Nach 3 h enthielt das Eluat 99,7 % Dimethylcarbonat.

## Patentansprüche

1. Verfahren zur Abtrennung von Methanol aus einem Gemisch von Dimethylcarbonat (DMC) und Methanol, in welchem der Methanolgehalt 0,01 - 40 Gew.-%, bezogen auf das Gewicht der Mischung beträgt, dadurch gekennzeichnet, daß ein solches Gemisch mit einem Ionenaustauscher in einer Menge von 1 - 100 % des Gewichts des Gemisches bei -50°C bis +100°C und 0,1 - 10 bar behandelt wird.

2. Verfahren nach Anspruch 1, dadurch gekennzeichnet, daß das zu behandelnde Gemisch 0,01 - 30 Gew.-% Methanol, bevorzugt 0,01 - 10 Gew.-% Methanol enthält.

3. Verfahren nach Anspruch 1, dadurch gekennzeichnet, daß der Ionenaustauscher in 5 - 50 % des Gewichts des zu behandelnden Gemisches eingesetzt wird.

4. Verfahren nach Anspruch 1, dadurch gekennzeichnet, daß bei -20 bis +100°C, bevorzugt bei 0 - 50°C gearbeitet wird.

5. Verfahren nach Anspruch 1, dadurch gekennzeichnet, daß als Ionenaustauscher ein Kationenaustauscher, ein Anionenaustauscher oder ein Gemisch beider eingesetzt wird.

6. Verfahren nach Anspruch 1, dadurch gekennzeichnet, daß die Behandlung mit einem Ionenaustauscher im quasikontinuierlichen Säulenbetrieb vorgenommen wird.

7. Verfahren nach Anspruch 5, dadurch gekennzeichnet, daß ein Kationenaustauscher eingesetzt wird.

8. Verfahren nach Anspruch 7, dadurch gekennzeichnet, daß ein mit Metallionen beladener Kationenaustauscher eingesetzt wird.

## Claims

1. Process for separating off methanol from a mixture of dimethyl carbonate (DMC) and methanol, in which the methanol content is 0.01-40% by weight, based on the weight of the mixture, characterized in that such a mixture is treated at from -50°C to +100°C and 0.1-10 bar with an ion exchanger in an amount of 1-100% of the weight of the mixture.

2. Process according to Claim 1, characterized in that the mixture to be treated contains 0.01-30% by weight of methanol, preferably 0.01-10% by weight of methanol.

3. Process according to Claim 1, characterized in that the ion exchanger is used in an amount of 5-50% of the weight of the mixture to be treated.

4. Process according to Claim 1, characterized in that it is carried out at from -20 to +100°C, preferably at 0-50°C.

5. Process according to Claim 1, characterized in that the ion exchanger used is a cation exchanger, an anion exchanger or a mixture of both.

6. Process according to Claim 1, characterized in that the treatment with an ion exchanger is carried out in pseudocontinuous column operation.

7. Process according to Claim 5, characterized in that a cation exchanger is used.

8. Process according to Claim 7, characterized in that a cation exchanger loaded with metal ions is used.

## Revendications

1. Procédé pour séparer le méthanol d'un mélange avec du carbonate de diméthyle (DMC) dans lequel la teneur en méthanol représente de 0,01 à 40 % du poids du mélange, caractérisé en ce que l'on traite ce mélange par un échangeur d'ions en quantité de 1 à 100 % du poids du mélange à des températures de -50 à +100°C et des pressions de 0,1 à 10 bar.

2. Procédé selon la revendication 1, caractérisé en ce que le mélange à traiter contient 0,01 à 30 % en poids de méthanol, de préférence 0,01 à 10 % en poids de méthanol.

3. Procédé selon la revendication 1, caractérisé en ce que l'on utilise l'échangeur d'ions en quantité de 5 à 50 % du poids du mélange à traiter.

4. Procédé selon la revendication 1, caractérisé en ce que l'on opère entre -20 et +100°C, de préférence entre 0 et 50°C.

5. Procédé selon la revendication 1, caractérisé en ce que l'on utilise en tant qu'échangeur d'ions un échangeur de cations, un échangeur d'anions ou un mélange des deux types.

6. Procédé selon la revendication 1, caractérisé en ce que le traitement par un échangeur d'ions est réalisé en quasi-continu sur des colonnes.

7. Procédé selon la revendication 5, caractérisé en ce que l'on utilise un échangeur de cations.

8. Procédé selon la revendication 7, caractérisé en ce que l'on utilise un échangeur de cations chargé en ions métalliques.
